## Europäisches Patentamt
## European Patent Office
## Office européen des brevets

(19)

(11) Publication number: **0 109 204**
**A2**

# EUROPEAN PATENT APPLICATION

(21) Application number: **83306358.9**

(51) Int. Cl.³: **A 61 B 6/02**

(22) Date of filing: **19.10.83**

(30) Priority: **15.11.82 US 441904**

(43) Date of publication of application: **23.05.84 Bulletin 84/21**

(84) Designated Contracting States: **DE FR GB NL**

(71) Applicant: **PICKER INTERNATIONAL, INC., 595 Miner Road, Highland Heights Ohio 44143 (US)**

(72) Inventor: **Mattson, Rodney A., 6532 Melshore Drive, Mentor Ohio 44060 (US)**
Inventor: **Brunnett, Carl, 1650 Mayfair Boulevard, Mayfield Heights Ohio 44124 (US)**
Inventor: **Flugan, David, 24455 Lakeshore Boulevard 1011E, Euclid Ohio 44123 (US)**
Inventor: **Manring, John M., 2565 Guilford, Cleveland Ohio 44118 (US)**
Inventor: **Zupancic, Anton Z., 10645 Hickory Hill Ct, Kirtland Ohio 44094 (US)**

(74) Representative: **Pope, Michael Bertram Wingate et al, Central Patent Department The General Electric Company, p.l.c. Hirst Research Centre East Lane, Wembley Middlesex HA9 7PP (GB)**

(54) **Method and apparatus for a modular detector mount in a computed tomography scanner.**

(57) In a computed tomography scanner, method and apparatus for mounting a plurality of detectors (98) in a position to measure x-radiation intensity from an x-ray source (40). The apparatus includes a module (50) for mounting a number of closely spaced detectors (98) in a circular array about a patient aperture (18). Each module (50) supports sixty closely spaced detectors (98) so that the entire array of 1200 detectors requires twenty such modules. In addition to the detectors, each module supports electronics (96, 94) for converting an analog signal from the detectors into a sequence of pulses where the pulse repetition rate is related to the x-ray intensity impinging on a detector. The modules can be readily removed from a stationary scanner gantry for service or replacement. When in place, the modules define a passageway (112) through which cool air is routed to cool the detectors (98) and thereby reduce their noise.

9-362                    <u>Description</u>

<u>Method and Apparatus for a Modular Detector</u>
<u>Mount in a Computed Tomograph Scanner</u>

<u>Technical Field</u>

The present invention relates to computed tomography and more particularly to an improved detector mounting scheme for use in a computed tomography (CT) scanner.

<u>Background Art</u>

The last decade has seen the practice of computed tomography mature from an experimental technique to an accepted diagnostic procedure used in hospitals throughout the world. The computed tomography diagnosing technique differs from conventional x-ray radiation technique in that an image representing density variations across a patient slice can be obtained using the computed tomography technique whereas conventional x-rays yield only a shadow image of the patient cross-section. A physician viewing a conventional x-ray must be truly skilled to determined differences in density between structures on the x-ray. The physician viewing an output from a computed tomography apparatus, however, can immediately discern differences in density from the image presented to him.

In computed tomography a patient or subject cross-section of interest is successively scanned from a multitude of orientations by an x-radiation source which directs x-rays through the patients slice of interest. One or more detectors positioned on an opposite side of the patient from the source obtain intensity readings of the x-radiation after it is passed through the patient. If enough intensity measurements or readings from a variety of orientations are obtained, these intensity readings can be utilized according to one of a number of known reconstruction algorithms to yield a density mapping of the patient cross-section. The reconstruction technique is derived from mathematical reconstruction algorithms dating back to the beginning of the twentieth century. Each intensity value of radiation passing through the patient corresponds to a line integral of an attenuation function taken through the patient cross-section from the source to the position the intensity is sensed.

A first generation of computed tomography devices were quite slow, and produced low resolution images of the objects being scanned. This first generation of machines, however, did validate the basic computed tomography techniques and suggested that with refinement the computed tomography procedure could be utilized to aid the physician in diagnosing internal structure of a patient.

Each first generation machine used a single source of x-radiation and a single detector which were caused to scan in a linear fashion past a subject a number of times at a number of different angular orientations. In operation the first generation machines used a linear scan technique whereby detector and source were repetitively and successively translated, then rotated and then retranslated to produce intensity readings suitable for analysis reconstruction. These first generation machines were slow. They could take more than ten minutes to obtain enough intensity readings to enable state of the art computers to generate a crude image. The slowness of the process and lack of resolution in the final image were serious concerns that had to be overcome before computed tomography could be routinely utilized as a tool for useful diagnosis.

A second generation of computed tomography devices operated on the same principle that the first generation developed but significantly increased resolution while speeding the time it took to provide a useable image.
A second generation device included a source of x-radiation and a bank or array of detectors which moved in unison with the source in a motion similar to the first generation machine i.e. translations and rotations were successively alternated to irradiate the patient from a number of orientations thereby producing enough intensity readings to reconstruct an image. It was the second generation of computed tomography apparatus which became commercially viable and was sold for use by hospitals in diagnosing patients.

A third generation of computed tomography apparatus resulted in even faster imaging. Third generation machines no longer required alternating translations and rotations to produce an image. The motion in the third generation machines is characterized as orbital wherein a single source and a bank or array of detectors orbits in unison about a patient. In a third generation CT machine time was no longer wasted in stopping and starting the source and detectors. The third generation computed tomography machines were much faster but suffered from deficiencies in data sampling and a so-called ring artifact. These deficiencies have been documented in the literature and need not be discussed at length except to point out that the enhancements and speed provided by the third generation device were substantially off-set by them.

Third generation computed tomography scanning systems are still being marketed as are CT devices built in accordance with a fourth generation design. The fourth generation design includes an array of detectors which are stationary with respect to a moving x-ray source. Typically the fourth generation array of detectors completely surround a patient aperture through which the patient is positioned for CT scanning. An x-ray source then irradiates the patient from a number of different orientations which is most typically achieved by orbiting or rotating the x-ray source about the patient. The fourth generation design eliminates the stopping and starting action common to the first and second generation systems so the speed advantages of the orbital type arrangement (third generation) are achieved and often exceeded and the ring artifact problem is not experienced. The time period required to produce a patient image in a typical fourth generation CT scanning system has been reduced to the order of a second and it is perceived that this time can be cut even further as computed tomography techniques are enhanced.

One feature common to all computed tomography systems is that they must include apparatus for detecting x-radiation intensity that has passed through the patient. In the first generation CT systems this apparatus involved a single detector which moved in unison with the moving source. The second and third generation CT systems included a plurality of detectors also moving with respect to the patient and in the fourth generation systems a high number are used.

Early computed tomography used one of two types of x-radiation detectors. One type detector is the scintillation detector which used a crystal for converting x-radiation impinging upon it into visible light which was then detected by a photomultiplier tube. This tube converted the light into an electrical current and amplified this current to a level suitable for measuring the intensity.

Gas ionization chambers were (and still are) also used in some CT designs. In such a chamber the high energy radiation passing from the source through the patient to the detector causes an ionization of the gas stored in the ionization chamber as it collides with gas molecules. By measuring the current generated in this ionization process, an indication of x-radiation intensity is obtained.

Photodiodes have increasingly replaced the photomultiplier tube for detecting light from a scintillation crystal utilized in a CT environment. These detectors typically comprises a scintillation crystal mounted in close proximity to a photodiode which produces an output current proportional to the x-ray intensity striking the scintillation crystal.

In a fourth generation environment, for example, an array of 600 or more closely packed photodiodes are positioned in a circular array to circumscribe a patient aperture so that x-radiation from a rotating x-ray tube impinges upon each of the detectors comprising the array as the x-ray tube orbits around the patient. Electronics coupled to each of the detectors converts the current output from each photodiode into a digital signal proportional to the x-ray intensity impinging upon a given detector.

The detectors have advantages over gas ionization or photomultiplier tube detectors. Perhaps the most significant advantage the solid state detectors have is the very close packing density these detectors permit in a fourth generation machine. A typical scintillation crystal and photodiode can be mounted in a package sufficiently small to allow well over one thousand detectors to be positioned about a circular array.

Each of the detectors in the array must be individually mounted to the CT apparatus and coupled to electronics for analyzing intensity readings from the detector. The logistics in keeping track of which detector is detecting which signal and in mounting the detectors when the number exceeds one thousand can be complex.

The typical computed tomography apparatus includes circuitry which converts an analog current passing through a photodiode into a digital signal. Circuitry associated with each of the detectors performs this function prior to the storing of digital representations of x-radiation intensity. As the packing density and number of detectors increases, it becomes increasingly difficult to mount and maintain the circuitry needed to analyze the current outputs from the photodiodes.

One proposal for mounting detectors in a CT environment is to divide the detectors comprising the CT array into groupings of eight. Each of the detectors is individually mounted to a detector holder which is then screwed into a locating block. The locating block supports eight individual detectors which then are ganged together to form the detector array. The proposal has a disadvantage in that each of the detectors comprising an array must be individually mounted into a mounting block prior to the arrangement of those detectors into groupings. Since only eight detectors comprise each of the groupings, an excess of 125 of these groupings are needed to construct a fourth generation computed tomography scanner of current standards.

The four detectors create a flat surface so that the proposal does not appear to be suitable for a circular fourth generation CT arrangement and would appear to be more particularly adapted to an orbiting scheme or even a translate rotate apparatus for a second generation device. While addressing the mounting problem, this proposal in no way suggests any technique for alleviating the electronics routing problem associated with handling the signals from one thousand stationary

7    0109204

detectors to electronics for analysing the signal outputs from the detectors and reconstructing an image.

Disclosure of the Invention

The present invention provides an orderly grouping of detectors in a computed tomography scanner. A detector mounting module both groups the detectors in an orderly arrangement and routes output signals from those detectors away from the module for subsequent signal processing. During construction of the CT scanning unit the modular arrangement allows each detector to be plugged into its associated module and then mounted to the computed tomography apparatus. The particulars of the mounting allow for great accuracy in positioning the detectors which is important in producing accurate intensity data. The modular design also has advantages in maintainance of the detectors. A module containing the discrete detectors can be removed from the scanner for testing. Testing need not perform the tests in an awkward inaccessible position.

In accordance with the invention a plurality of x-radiation detectors are mounted in closely spaced positions onto a curved substrate. Each detector is then coupled to circuitry also mounted to this substrate for converting analog outputs from the detectors into pulses. To form a stationary detector array, a plurality of the substrates are then mounted to a fixed support to form a segment of a circular array of detectors. In a preferred embodiment of the invention, the array completely circumscribes a central region of interest to form a circular array. In use, an x-radiation source mounted inside the array selectively irradiates the detectors comprising the circular array as it moves in a circle around a subject of interest.

In a preferred design, the curved substrate comprises a so-called motherboard of plastic printed circuit material. The circuitry for processing the output signals from each of the detectors on the motherboard is mounted to a number of daughterboards mounted to the motherboard and including connectors which couple with associated connectors on the motherboard for transmittal of signals to and from the daughterboards and the motherboard. Each daughterboard corresponds to one and only one detector so that if a problem is discovered with the output from a one detector not only can that detector be easily removed from the motherboard but the circuitry for processing the analog output of the detector can also be easily checked and/or replaced.

Both the motherboard and its associated daughterboards are all mounted in a box-like container which provides electrical shielding of detectors and electronics. The box-like container has two self aligning threaded connectors which engage threaded holes in a scanner gantry. To mount a first of the modules in place, the assembler aligns the threaded connectors with the threaded holes and screws the module in place with the aid of a pair of knurled knobs. At the side of each module is defined a tongue and groove pair which orients the next module as it is mounted to the stationary gantry. Each modular subassembly is mounted in place until the entire circular detector array has been mounted. A preferred design includes twenty modules with each module mounting sixty detectors.

The detectors themselves are packaged in a unique way. A scintillation crystal is optically bonded to to a photodiode and the two are then packaged in a light seal which prevents stray light from enering the package. An aluminum can provides mechanical protection and an electrical shield. These photodiodes are inserted one-by-one into the motherboard which then is precisely positioned with respect to the box-like module. A defective detector can be removed quite simply without the need to disrupt the remaining detectors in the module array. The end most detector of each array abut the end most detectors of the next closest modules to avoid gaps between modules which might result in artifacts in the resulting computed tomography image.

The modules define a passageway through which cooled air can be forced to cool the detectors as well as the electronics mounted to the mother and daughter boards and each module. To accomplish the air conditioning, certain ones of the module structures include an inlet through which cool air can be forced. Remaining ones of the array define closed structures which allow the detectors to be maintained at sub-ambient temperature during operation of the computed tomography apparatus.

From the above it should be appreciated that one object of the present invention is the design of a modular array for positioning detectors in a computed tomography environment. The design greatly facilitates manufacturability of the computed tomography apparatus as well as allowing ease in maintaining that apparatus once it is installed. These and other objects, advantages and features of the invention will become better understood when a preferred embodiment of the invention is described in conjunction with the accompanying drawings.

Brief Description of the Drawings

Figure 1 is a perspective view of a computed tomography (CT) scanner of the stationary detector design and the peripheral components of that scanner;

Figure 2 is a front elevational view of the CT scanner schematically depicting the major components including an air conditioning system;

Figure 3 is a perspective view of detector modules in relation to a stationary scanner gantry;

Figure 4 is an elevation view of two modules mounted to the gantry;

Figure 5 is a sectional view taken along the line 5-5 in Figure 4;

Figure 6 is a side elevational view of a compressor in the air conditioning system;

Figure 7 is a top plan view on an enlarged scale of one of the detector mounting modules;

Figures 8 and 9 are front and rear elevation views on the seal of Figure 7 of the detector mounting module;

Figure 10 is a bottom plan view with parts broken away to reveal internal structure, of the detector module;

Figure 11 is an end elevational view of the detector mounting module;

Figure 12 is a sectional view taken along the line 12-12 of Figure 10;

Figure 13 is an enlarged view, partially in section, showing the the mounting of an individual detector in the detector mounting module;

Figure 14 is a partially sectioned elevational view, on an enlarged scale, of an individual x-radiation detector;

Figure 15 is a top plan view of the Figure 14 detector;

Figure 16 is an end view of the Figure 14 detector; and

Figures 17A-C are schematics of the electronics mounted to the mother and daughter printed circuit boards comprising each detector module.

Best Mode for Carrying out the Invention

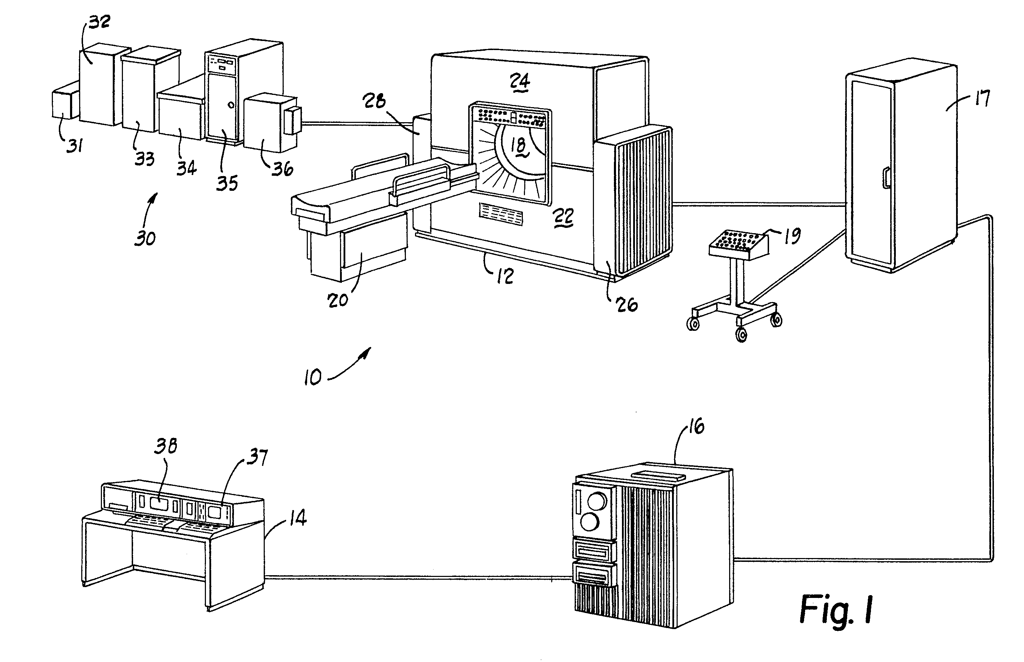

Turning now to the drawings, Figure 1 illustrates a computed tomography scanning system 10 used in imaging cross-sectional slices of interest in a patient. The computed tomography system 10 comprises a scanner 12, a viewing console 14, a computer 16, and specialized electronics 17 needed by the scanner 12 for control and data handling.

The scanner 12 is a fourth generation computed tomography scanner where a fixed array of detectors surrounds a patient aperture 18. During imaging a patient is positioned on a couch 20 and then moved into and through the patient aperture 18 until a cross-sectional slice to be imaged is appropriately positioned. A scanner front panel is divided into two portions 22, 24 which are hinged to the scanner housing. These two portions swing away from the positions they are shown in Figure 1 to allow the interior of the scanner 12 to be accessed. The scanner housing is supported by a pair of supports 26, 28 and can be tilted about an axis extending through the supports parallel to the floor. In this way, patient cross-sections other than a vertical cross-section can be obtained without repositioning the patient.

The generation of x-radiation requires a series of electronic subsystems 30 shown to the side of the computed tomography scanner 12. In an x-ray tube, highly accelerated electrons are directed to a tube anode from a cathode and in particular electrons having nearly 150,000 electron volts of energy strike the anode to produce x-radiation. To accelerate the electrons, a voltage of nearly 150,000 volts must be transmitted from the electronics 30 to rotating portions of the scanner 12. The electronics needed to generate these high voltages includes a heat exchanger 31, a power module 32, a high voltage transformer 33, a voltage regulator 34, an x-ray control module 35, and a high speed starter 36.

In computed tomography scanning, special electronics needed to analyze intensity values as detected by the scanner 12 are also required. This specialized electronics 17 counts output pulses from the scanner detectors as well as controls movement of an x-ray tube and coordinates this movement with the analysis of the output signals. A service module 19 coupled to the specialized electronics 17 allows the scanner 12 to be tested without the aid of the computer 16 or the viewing console 14.

High speed computed tomography scanning is possible only through use of a high speed data processing computer 16. The illustrated and presently preferred computer 16 is a 32 bit Perkin-Elmer mini computer with a disc storage capacity of 320 million bytes. This computer 16 performs the sophisticated data processing for reconstructing a grid-like mapping of density variations inside the patient slice from intensity readings taken from a plurality of detectors surrounding the patient aperture. The particular computer chosen is responsible for not only analysing and reconstructing cross-sectional image densities but also for displaying this information on a console 14.

The console depicted in Figure 1 includes a first work station 37 for a technician operating the computed tomography apparatus and a second work station 38 for a person responsible for diagnosing the images produced. Although not shown in Figure 1, a remote diagnostic station is optionally provided so that the person diagnosing the patient need not be in the same location as the operator.

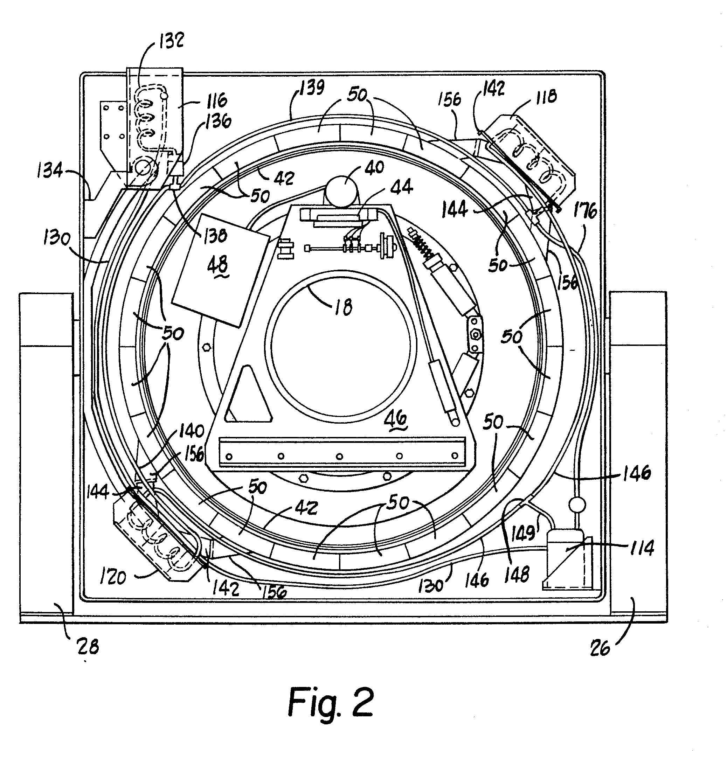

When the panel portions 22, 24 are pivoted away from the position shown in Figure 1, an x-ray tube 40 and fixed array 42 of x-radiation detectors are visible (see Figure 2). The illustrated array 42 completely encircles the patient aperture 18. During scanning, the x-ray tube 40 generates an x-ray beam which is shaped by a collimator 44 to provide a generally planar spread beam of radiation that passes through the aperture 18 to opposed detectors in the array 42. The x-radiation is attenuated by the patient (or any other object in the path of the x-radiation) and its intensity is then detected by detectors in the array 42. The tube 40 and collimator 44 are both mounted to a CT frame 46 which is rotatably mounted to the scanner 12. A motor (not shown) at the rear of the CT apparatus imparts rotational motion to the frame 46 which is journalled on stationary portions of the scanner 12 by a bearing (not shown) which surrounds the patient aperture.

The x-ray tube 40 heats during operation and in particular, unless it is cooled, the tube 40 can overheat and malfunction. For this reason, a cooling unit 48 is mounted to the side of the x-ray tube and coupled to the tube to circulate coolant to the tube housing.

The array 42 of detectors is made up of individual modules 50 which each support a plurality of closely spaced detectors in fixed relation around the patient aperture 18. In accordance with a preferred design, each module 50 supports sixty detectors so that the 20 modules comprising the array 42 support 1200 detectors in a circular array.

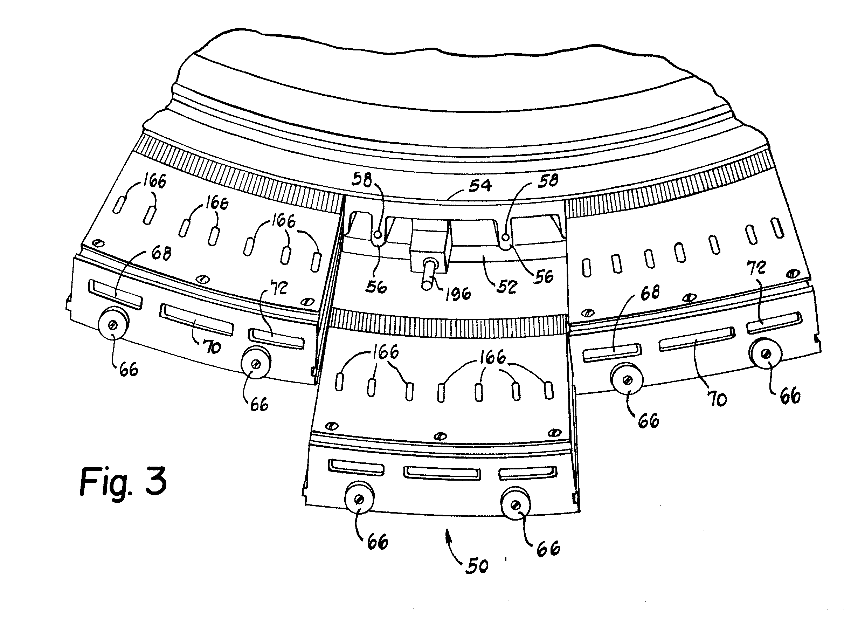

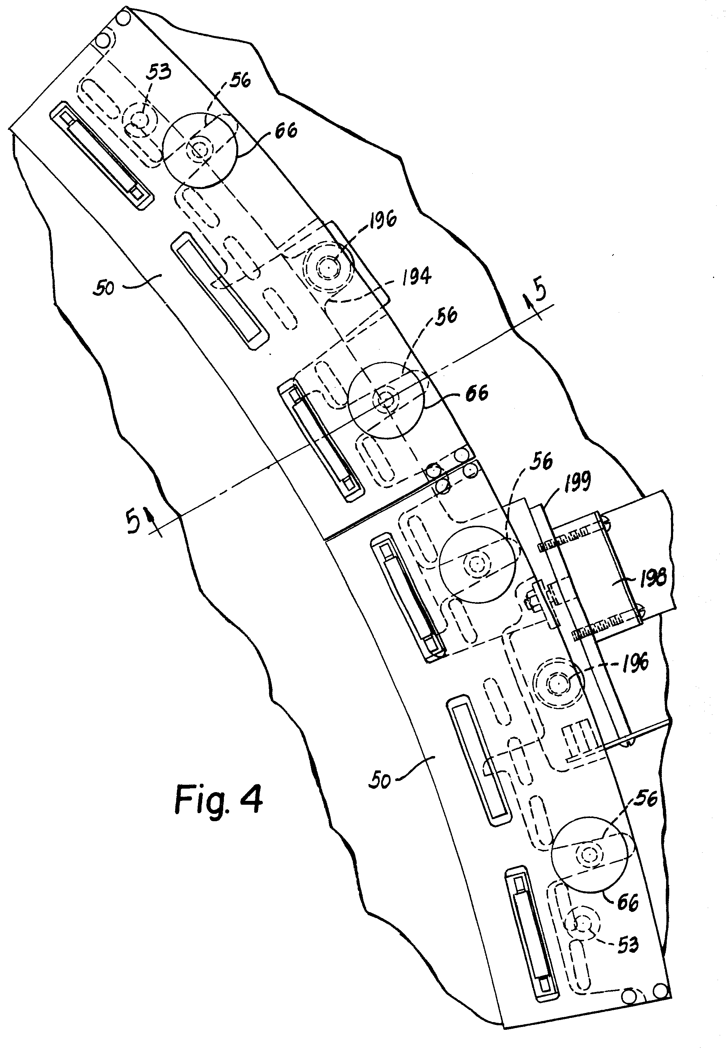

Each of the modules 50 can be easily mounted to or removed from the scanner 12 to facilitate assembly or maintenance of an assembled unit. A stationary gantry 52 supports through a connector 53 a number of module mounts 54 (see Figures 3, 4 and 5), with each module mount 54 defining a series of fingers 56 which extend radially outward from the module mount 54. These fingers define threaded openings 58 into which threaded rods 60 on the modules 50 are screwed to mount the modules 50 about the patient aperture 18. During assembly, a first module is screwed into place and other modules 50 comprising the detector array are successively mounted to a module mount 54 in coacting relationship with the first module until an entire array of twenty modules has been mounted in place.

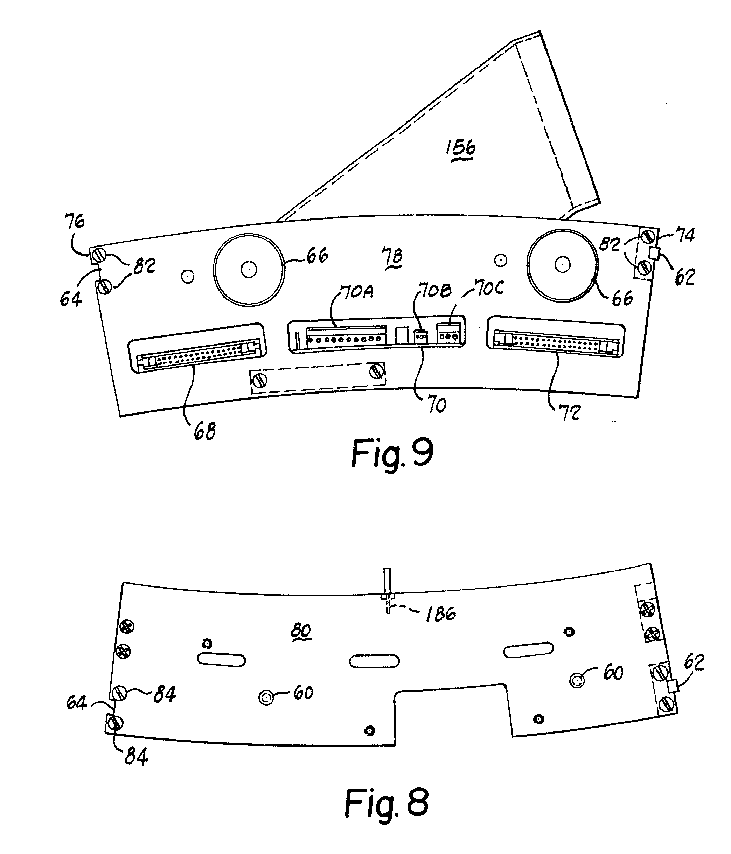

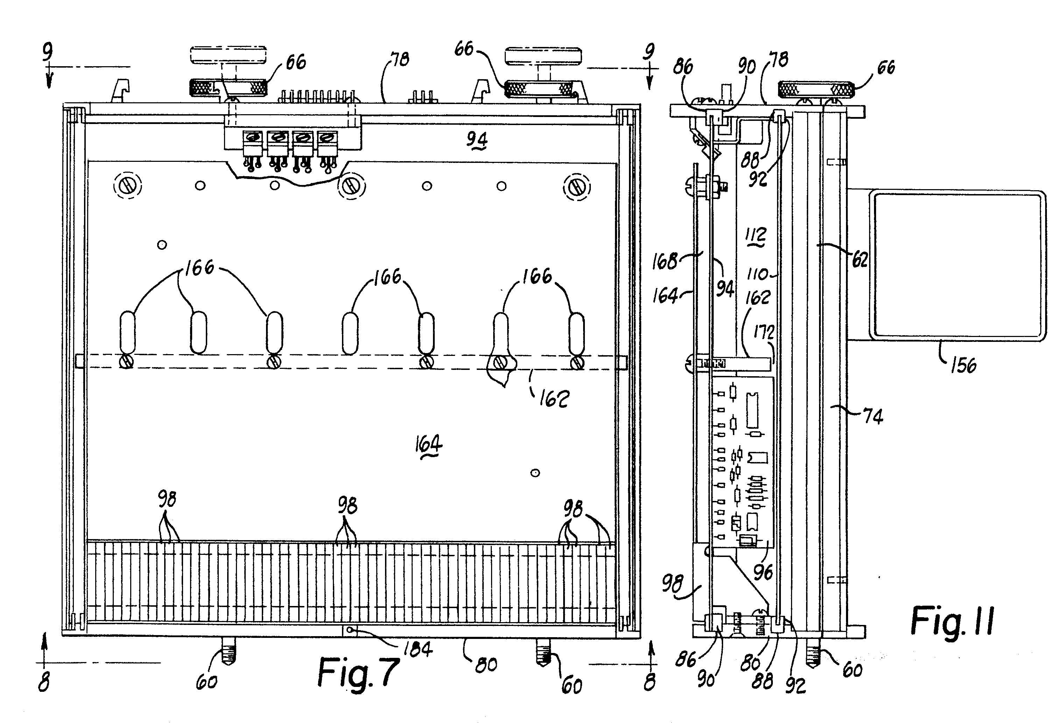

The sides of each module 50 respectively include a tongue 62 and a groove 64 (Fig. 8) to help position the module 50 as it mounted. Once a first module is in place, subsequent ones of the modules 50 are inserted by interlocking the tongue 62 and groove 64 of adjacent modules together and sliding the module into place until the rods 60 engage the threaded openings 58 on the module mount. Each rod 60 terminates in a knurled knob 66 which allows the modules to be hand tightened to the gantry. As each of the modules 50 is connected to the gantry 52, ribbon connectors are attached to three sockets 68, 70, 72 on the module (see Figure 9). These ribbon connectors route signals to and away from their module 50 and in particular two of the sockets 68, 72 have pins which route pulses corresponding to x-ray intensity from the multiple detectors of their module 50.

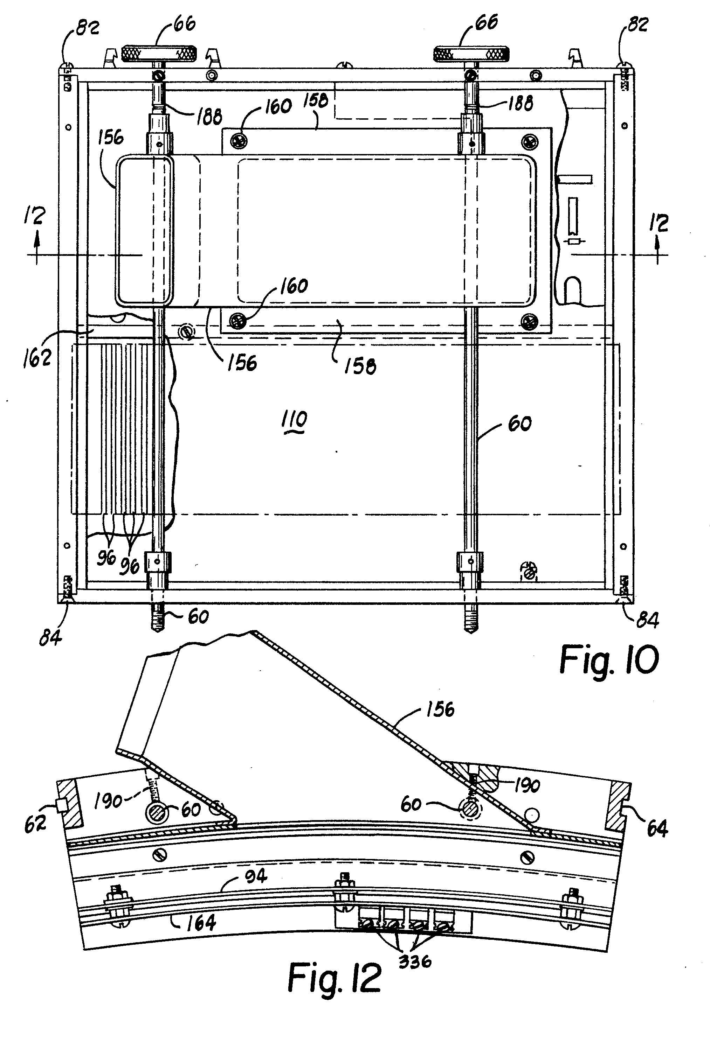

The shape of each module is defined by two end walls 74, 76, a front wall 78, and a back wall 80 which in conjunction define an arcuately curved box-like structure into which sixty detectors are mounted in a circular arc. The walls are joined by threaded connectors 82, 84 (see Figure 10). It should be noted that the connectors 84 used to couple the back wall 80 to the two end walls 74, 76 comprise countersunk    flat head screws so that when the threaded rods 60 mate with the threaded openings 58 in the module mount 54, each module 50 can be tightened down flush with the front surface of this mount 54.

The front and back walls 78, 80 define two pairs of grooves 86, 88 (Figure 11) through which two pairs of trackways or guide members 90, 92 extend across the entire width of each module 50. A first of the guides 90 positions a large printed circuit board 94 inside the module 50. Each guide 90 is curved to conform to the curvature of the module so that the printed circuit board 94 also follows the curvature of this module. (See Fig. 12) Socketed into this large so-called motherboard 94 are a plurality of smaller so-called daughterboards 96 each coupled to a single detector 98. In the preferred embodiment the purpose of the daughterboards is to receive signals from one of the detectors, convert an analog output from this detector into a sequence of digital pulses where the pulse repetition rate of the output corresponds to the intensity of the x-radiation impinging upon that detector. Alternately the daughterboards could include some or all of the circuitry to amplify, filter, integrate, sample and hold the analog signal of the photodiodes.

The daughterboards are electrically shielded one from the other by thin metal sheets which are attached to one side of each daughterboard. These shields are electrically grounded.

The second pair of guide members 92 positions a sheet metal plate 110 outside the motherboard to cover both mother and daughterboards and enclose them so that the detectors 98 and their associated electronics are electrically shielded. This sheet 110 also helps define a passageway 112 through which cool air is forced to cool both the detectors 98 and the electronics for analyzing analog signals from the detectors. The lowering of temperature of both the detectors and the circuitry coupled to those detectors reduces the noise of the electric outputs from these components which in turn

0109204

enhances the signal to noise ratio which results in an improved CT image.

The major components of an air conditioning system for directing cool air to the detectors 98 are seen most clearly in Figures 2 and 6. This air conditioning system comprises a compressor 114, a condensor 116 and two evaporator units 118, 120. Since the detectors in the disclosed fourth generation CT apparatus are all stationary, the cooling system components are also all stationary and in particular are mounted on the four corners of the CT housing between that housing and the detector array 42.

The air conditioning system utilizes a freon coolant to accomplish a heat exchange between cold freon and the relatively warm ambient air surrounding the CT scanner 12. As a first step of the cooling process, the compressor 114 compresses freon in a gaseous state to an elevated temperature and directs this compressed freon from an output 122 (Figure 6) along a 3/8 inch copper tube 124 to a 3/8 tee 126. On one side of this tee 126 is a hot gas bypass valve 128 to be discussed further. The other side of the tee directs hot compressed freon along a 3/8 inch copper feedline 130 around the periphery of the CT detector array 42 to the condensor unit 116.

At the condensor unit, the compressed freon is allowed to expand and therefore cool. Condensor coils 132 inside the condensor unit 116 heat ambient air as it is forced past the condensor coils and exited from the system by a hot air exhaust 134. As the liquid freon leaves the condensor 116, it passes through a filter 136 which purifies the liquid freon. The output from the condensor 116 leads to a 1/4 inch tee 138 which divides the freon into separate paths 139, 140 to the two evaporator units 118. Inside the evaporator units the liquid freon evaporates, and as it does so cools air which is blown from the back of the computed tomography unit through the condensor units 118, 120 to a pair of outlets 142, 144 on each of the evaporator units.

The gaseous freon is now routed back to the compressor for recycling. An outlet from each of the evaporator units directs the freon along a 3/8 inch copper pipe 146 to a 3/8 inch tee 148 in the vacinity of the compressor. This tubing 146 is insulated before installation onto the CT unit as is a 3/8 inch copper tube 149 leading from the tee 148 to an accumulator tank 152. From the accumulator tank 152, the freon is routed back to the compressor along a 3/8 inch diameter copper tube 154 which is again insulated before installation.

Cold air from the evaporator units 118 exits the outlets 142, 144 from these units and passes through an air shroud 156 coupled to four of the detector modules 50 in close proximity to the evaporator units 118. As seen in Figure 2, only four of the modules 50 include this shroud 156. The remaining modules 50 are sealed along their exterior surface by the metal sheet 110. Each sheet 110 on those four modules which include the shroud 156 has openings stamped at appropriate locations to receive the shrouds 156. Each shroud 156 defines a lip 158 (Figure 10) which is coupled to the sheet metal 110 by suitable connectors 160, such as self tapping screws. .

The fans (not shown) which force the air through the evaporator units 118 also continue to force the cooled air through the shrouds 156 into the interior of the modules 50. This cool air enters the modules 50 and passes through a module passageway 112. With all 20 modules 50 in place, the passages collectively completely circumscribe the patient aperture. Each passageway 112 is defined by the metal sheet 110, the motherboard 94, the front wall 78, and an intermediate wall 162 coupled to the motherboard 94.

Also connected to the motherboard 94 is a shield 164 which as seen in Figure 7, includes a series of regularly spaced apertures 166 which align with a similar set of apertures (not shown) in the motherboard. Air circulating through the passageway 112 passes through the apertures in the motherboard to a second passageway 168 between the motherboard 94 and the shield 164. This second passageway 168 allows cool air to pass from the first passageway 112 to the vicinity of the plurality of daughterboards 96.

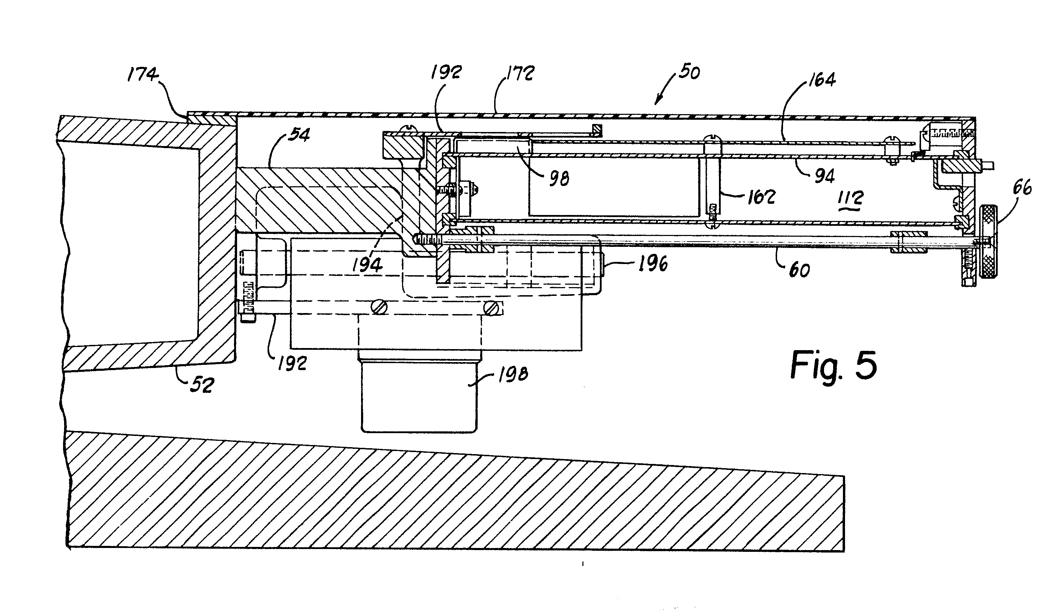

The holes 166 and the shield 164 allow cool air to pass through the shield through yet another passageway 170 defined by the front wall of the modules and a clear plastic sheet 172 which extends from the front wall of each module to an air seal 174 mounted to the stationary gantry 52 (see Figure 5). The preferred seal 174 comprises a double layer of foam tape applied to the gantry. The preferred sheet 172 comprises a number of segments which are bent and popped into place around the inside radius of the detector array. As seen in Figure 11, the intermediate wall 162 does not extend to the sheet metal plate 110 so that a gap 176 exists between the two.

The air flow paths for the cooled air from the evaporator units 118 can now be summarized. Cool air enters the shroud 156 to the passageway 112 and circulates about the interior of the module array. From the passageway 112, the cool air flows through the gap 172 to the vicinity of the sixty daughterboards 96 of each of the modules as well as passing through holes in the motherboard 94 to the second passageway 162 so that the cool air also contacts the daughterboards through this second passageway. The second passageway abuts the detector array made up of individual detectors 98 and in addition, the cool air passes through the apertures 166 in the shield 164 to contact a top surface of each detector.

The detector array is maintained at a temperature above the dewpoint to avoid water condensation on the detectors. The temperature modification takes into account the fact that the pressure of the freon entering the evaporator is related to its temperature and can be determined from a Mollier diagram for freon. As noted above, the air conditioner includes a bypass valve 128 which is pre-set to open when a certain pressure differential exists on the tubing leading to that valve. One side of the valve 128 is coupled to the compressor 114 through the tee 126 and the otherside of the valve 128 is coupled to copper tubing 176 connected to the evaporator units 118.

Whenever the pressure difference between the gas in the compressor and the liquid freon in the tube 176 exceeds the predetermined amount, the valve 128 opens forcing hot compressed freon into the cool liquid freon thereby raising the temperature of the liquid freon. In this way, the temperature of the freon reaching the evaporator units 118 never falls below a predetermined point and in particular, never falls below a point which will cause water to condense on the detectors. Use of the bypass valve 128 allows the compressor 114 to be continuously on during computed tomography scanning. In otherwords, the compressor need not cycle on and off to maintain the temperature of the detectors at a predetermined point, but instead, controls the temperature of the freon reaching the evaporating units 118 by the disclosed hot gas bypass valve 128.

The compressor 114, condenser 116, and two evaporator units 118, 120 are commercially available from Mc-Lean Engineering Lab of Princeton Junction, New Jersey. The blower for the evaporator units and the condensor is also available from McLean and is designated by part No. 30-4019-01.

Each of the detectors 98 has two pin contacts 178 which are inserted into sockets 180 in the motherboard 94. One of the pins is coupled to the shield and coupled to an anode of a photodiode 212 inside the detector 98. A second pin transmits an analog signal from the photodiode 212 to the socket 180 on the motherboard to its associated daughterboard by an edge connector coupling the daughterboard.

It is important that the side-to-side spacing of the detectors 98 comprising the detector array is accurate so that the intensity signals can accurately reconstruct a cross-sectional slice of the patient. For this reason, during construction of each module, the motherboard 94 must be very accurately aligned with respect to the walls which define the module. During assembly, the motherboard 94 is slid into its associated track 90 until a hole in the motherboard along its edge closest to the gantry 52 aligns with a hole 184 (see Figure 7) extending from the top of the back wall 80 to the point where the motherboard 94 coacts with this wall 80. When the hole in the wall aligns with a hole in the motherboard, a pin 186 (Fig. 8) is inserted into the two holes to insure the motherboard 96 remains properly aligned so that the position of the multiple detectors 98 comprising the array is precisely known and used in the reconstruction technique.

When a particular module 50 is to be removed from the detector array, the knurled knobs 66 are unscrewed and the particular module to be removed is disengaged

0109204

from the rest of the array by sliding it away from the gantry. Once the module has been removed, it is then advantageous to insure that the pointed end of the threaded rod 60 is retracted so that it does not come in contact and scratch critical surfaces on the CT apparatus. As seen in Figure 10, the rod 60 has a circular groove 188 in its circumference at a location slightly displaced from the knurled knob 66. By pulling the rod 60 away from the module 50 to a position shown in phantom in Figure 7, this groove 188 approaches the front wall 78 of the module 50. As movement continues, a ball plunger 190 (Fig. 12) aligned with the rod position engages the groove 188 preventing further movement of the rod until the plunger 190 is manually disengaged.

The disclosed CT scanner includes a series of ten aperture strips 192 (Figure 5) for partially blocking the radiation from reaching the detectors of the array 42. Each strip comprises a series of equally spaced slots in a lead, tungsten, or tantalum sheet which are movable between a position where no radiation is blocked to a position where a portion of each detector is shielded from radiation.

The shutter 192 is mounted to an aperture support 194 which in turn is supported by a guide rod 196 connected to the detector module mount 54. A bearing between the rod 196 and support 194 allows a shutter motor 198 to position the shutter with respect to the detectors supported by a given module mount 54. The motor 198 is supported by a plate 192 such that a gear (not shown) coupled to a motor shaft engages a second gear on the aperture support 194. The motor 198, at times when scans are not being conducted, drives the support and connected shutter 192 until it has reached a desired position (either retracted or one of two positions over the detectors 98).

0109204

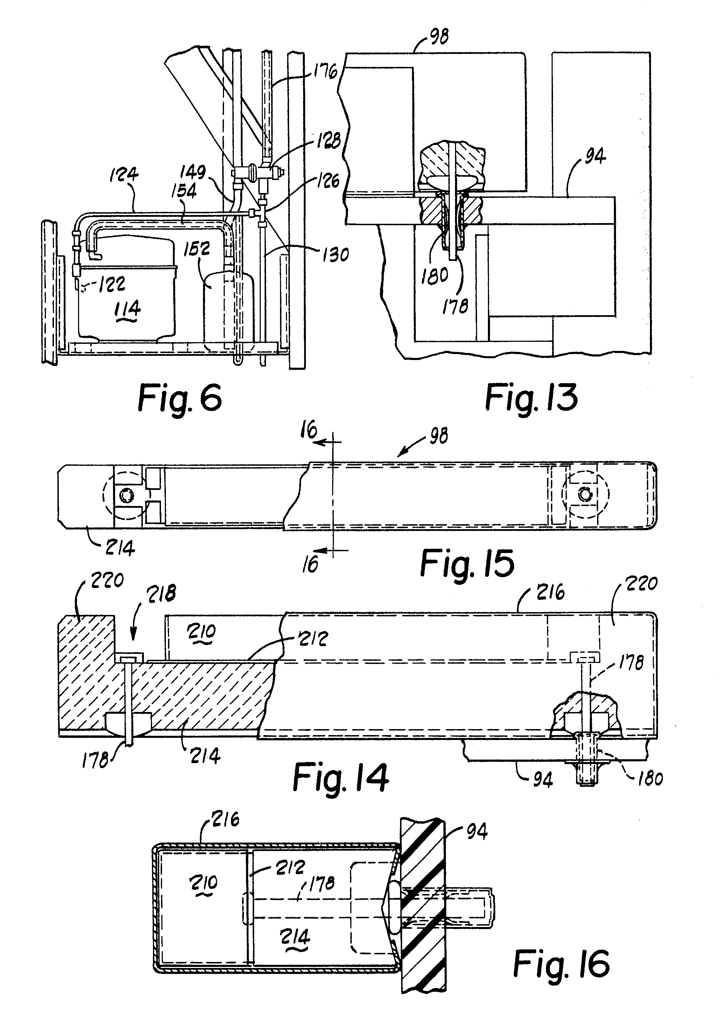

The particulars of the construction of each detector 98 can be seen most effectively in Figures 14-16. The detector 98 comprises a scintillation crystal 210 coupled to a photodiode 212 which in turn is mounted to a ceramic mounting block 214. The detector is enclosed in an aluminum can 216 which allows x-radiation to be freely transmitted to the scintillation crystal 210. In operation, the x-radiation from the x-ray tube impinges upon the scintillation crystal which converts the x-radiation into visible light which in turn affects the current flow in the photodiode 212. Changes in current produced by the x-radiation are converted from an analog signal into a sequence of pulses which can be counted.

Construction of the detectors 98 includes the following sequence of steps. As a first step, the crystal 210 is bonded to the photodiode 212. A preferred crystal is made from Cadmium Tungstate. The large area photodiode used in the preferred embodiment has a low capacitance, high shunt resistance and is mounted to the ceramic block 214. The crystal and diode are coupled together with a clear optical cement which in the preferred embodiment comprises a lens bond material available from the Eastman Kodak Company of Rochester New York under product No. HE80. As a next step, the photodiode and crystal are both cleaned and bonded together and placed in a mounting fixture, which is then placed in an oven and heated for four hours at 80°C. The operation of both the photodiode and crystal are tested both before and after the bonding process and then a white highly reflective paint is applied on a top and side surfaces of the scintillation crystal.

As seen in Figure 14, a gap 218 exists between the scintillation crystal 210 and two legs 220 extending from the ceramic mounting block 214. This gap allows a contact to be made between the photodiode 212 and the pin 178 which is inserted into the socket 180 in the motherboard 94. The photodiode is coupled to the pin 178 by a doubled wire bond of gold wire.

Once the leads have been attached to the pin, the scintillation crystal 210 is covered with a thin (approximately .002 inch thin) black vinyl tape to both seal the photodiode from light and also hermetically seal the package from contamination. The vinyl tape is then covered in a metal aluminum can 216 which extends completely around the tape and is bent back under the bottom surface of the ceramic mounting block 214. One of the pins 178 of the photodiode is also coupled to the aluminum can as well as a ground connection on the motherboard 94. Each detector 98 is individually inserted into the motherboard 94 until all sixty detectors for a given module are in place. The module is then mounted as described above.

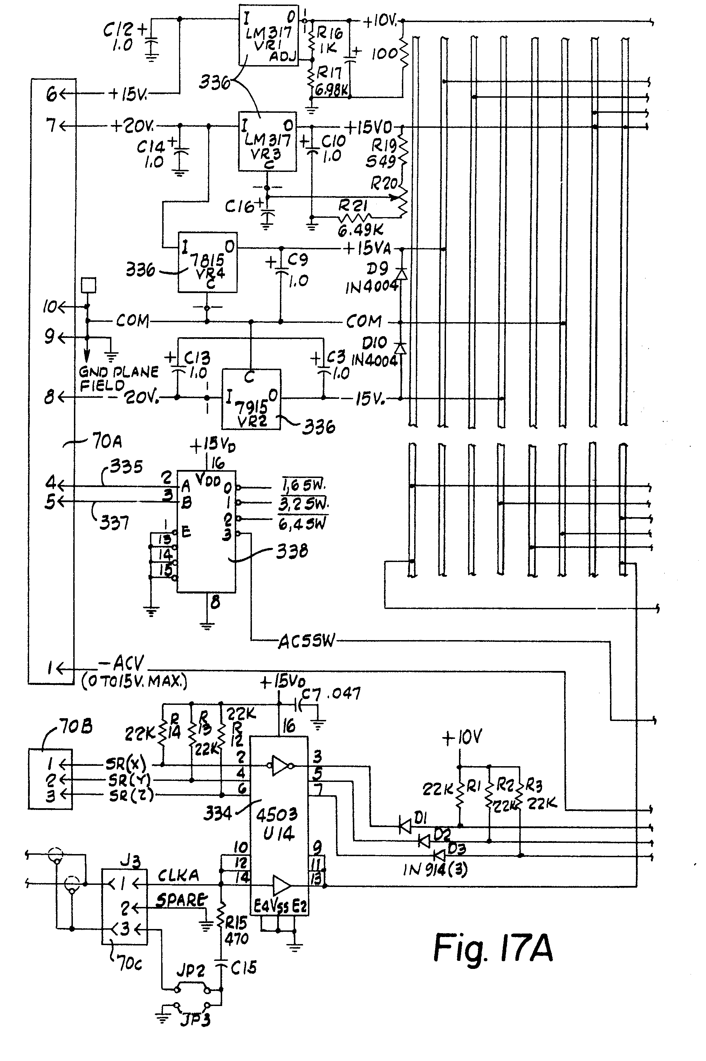

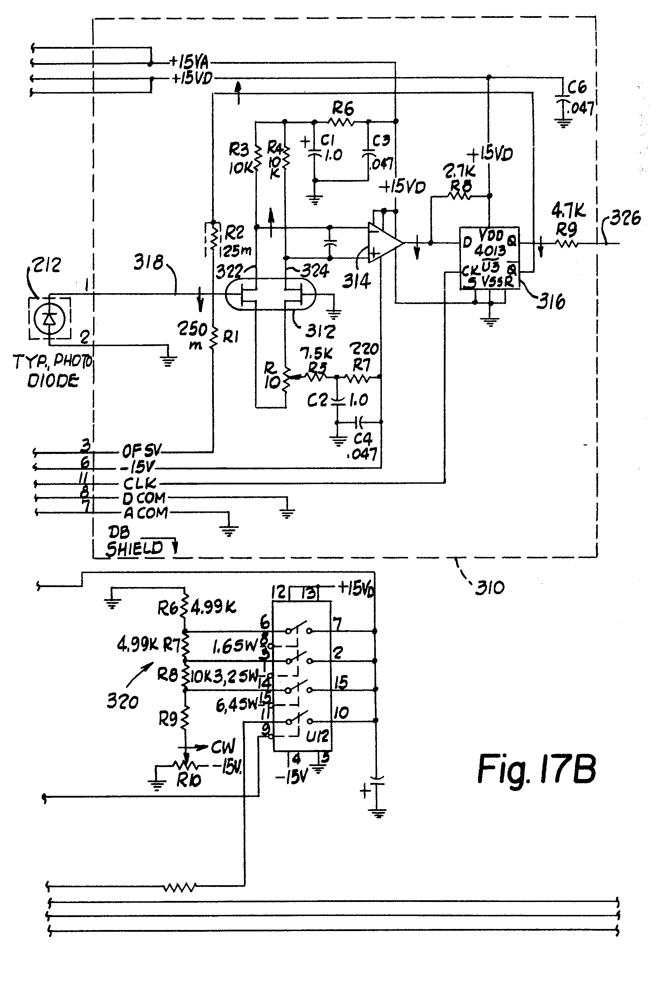

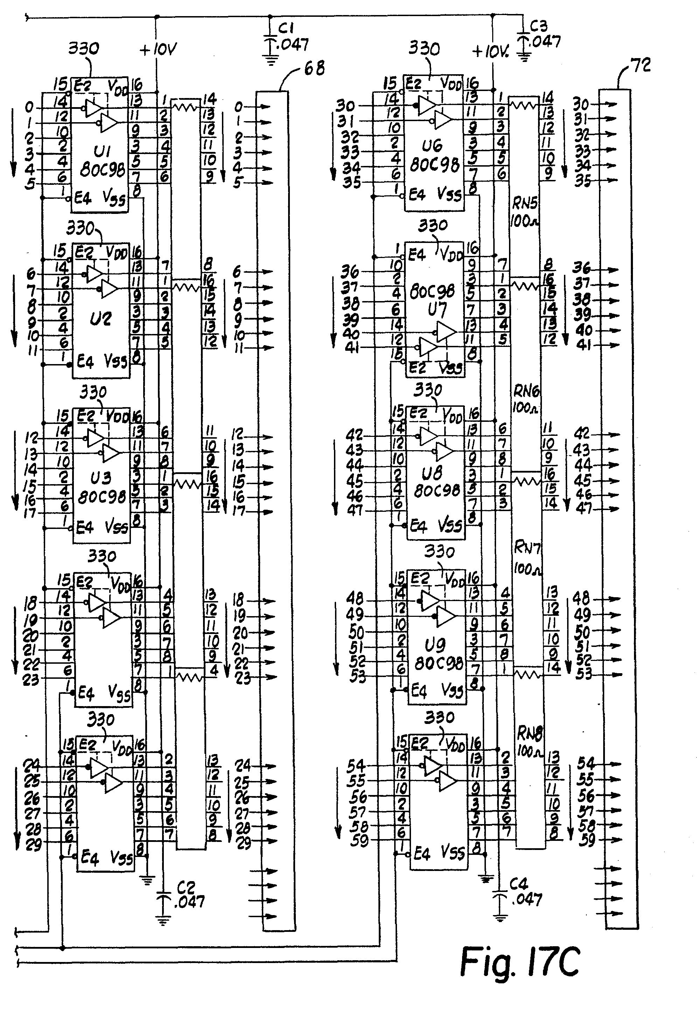

An electrical schematic for the circuitry on the mother 94 and daughter 96 boards is shown in Figures 17A-C. The electronics mounted to these boards converts the output from a photodiode into a series of pulses where the pulse repetition rate corresponds to the intensity of x-radiation impinging upon a given detector. It is appreciated that the circuitry for only one of the sixty daughterboards mounted to a given module is shown in these Figures.

The daughterboard 94 supports a current-to freq. converter i.e., its input is current and its output is a pulse train whose frequency is proportional to the input current. It is a closed loop system in that the current removed from the input junction is replaced by a series of feedback current pulses (unit charges) whose average value is equal to the current being removed.

The offset current and/or radiation induced current at an input 318 (from the photodiode) produces a negative charge on the input junction capacitance. This capacitance is made up of the junction capacitance. Of the photodiode 212 (major part), the "Miller" input capacitance of a preamplifier 312 and stray wiring capacitance.

A negative charge results in a negative voltage on the input capacitance. The preamplifier 312 amplifies this negative voltage and feeds it to a comparator 314 causing it to change state. This change of state is fed to the input of a "D" flip-flop 316. At the next clock pulse occurring after the comparator chage of state, the "D" flop is "set" causing the $\overline{Q}$ output to go positive. Because the $\overline{Q}$ output is connected to a 25 Megohm feedback resistor, a positive current flows into the input junction capacitance.

This positive current is large enough to change the charge on the input junction capacitance to a positive value within a clock period (luS). The preamp 312 senses the resulting positive voltage which in turn returns the comparator and the "D" flop input to their original states before the next clock pulse occurs. This next clock pulse then resets the "D" flop which in turn removes the positive voltage to the feedback resistor. The net result is that a "unit" charge has been fed back to the input capacitance forcing its voltage positive. The clock then waits until the input currents once again force the input charge (and voltage) negative whereupon the process repeats.

The "unit" charge is defined as $I\Delta T$ where I is Vd/Rfb and $\Delta T$ is the time between successive positive transitions of the clock input (luS). Each time a "unit" charge is fed back an output pulse is generated.

An output 326 from the daughterboard is coupled to one of six inputs on one of ten buffer units 330 socketed into the motherboard. The ten buffer units 330 thus can receive the sixty outputs from the sixty daughterboards mounted to each module. These buffer units 330 transmit the pulses from the associated daughterboard to a circuit (not shown) which counts the pulses and thereby generates a representation of the intensity output from a given photodiode comprising a given detector. A suitable circuit for counting the pulses is shown and described in U.S. Patent No. 4,052,620 to Brunnett which is assigned to the assignee of the present disclosure and incorporated herein by reference. Signals from the buffers 330 are transmitted to the two connectors 68, 72 on the front of the module 50 for transmission to this counting circuit.

It should be recalled that the computed tomography apparatus comprises 1200 detectors spaced in a circular array around the patient aperture. At any given time, however, the beam of x-radiation from the x-ray tube is irradiating approximately 1/4 of those detectors so the output from only 300 of the detectors is sampled at a given time.

In accordance with the preferred design, each of the output buffer units 330 is enabled by one of three inputs labeled SRX, SRY and SRZ in Figure 17A. When activated by one of these three inputs, a group of twenty output signals from the buffer units 330 is transmitted to its associated connector 68, or 72.

As the x-ray tube sweeps around the patient aperture and approaches the detectors of a given module, a first selector signal activates or enables a first group of twenty buffer outputs. As the x-ray tube continues to rotate and the x-radiation from the tube covers more of the detectors on the module, the other two selector inputs are activated causing forty and then all sixty outputs from the buffer units 330 to be activated and transmitted to the connectors 68, 72. After the x-ray tube has radiated that group of detectors, i.e., after the x-radiation passes the detectors comprising a given module, the selector inputs are sequentially turned off so that other output buffers on other modules can be accessed. In addition to transmitting the selector inputs to the buffer units 330, a different buffer 334 (Fig. 17A) also transmits an input comprising a one megahertz clock signal to a given motherboard. This clock signal is transmitted to a clock input on the D-type flip-flop 316 to reset that flip-flop at equal time intervals.

A plurality of reference signals are also routed to each motherboard through the connector 70. In particular, this connector transmits a 15 volt, 20 volt, ground, -20 volt and two offset select signals 335, 337 to the motherboard. A sequence of four voltage regulators 336 regulate the voltage inputs to and in particular they generate voltages of +10, +15 and -15 volts. The offset selections are coupled to a decoder 338 which converts the two inputs to the decoder into one of four outputs to select one or four of the offset voltages generated by a voltage divider 320. The choice of which offset voltage to choose depends on the intensity of the x-ray voltage and the speed with which the x-ray tube rotates. These factors determine which offset voltage will insure an adequate repetition rate from the comparator 314.

As noted above, the output signals from the buffer units 330 are counted to obtain an indication of the x-radiation intensity impinging upon each of the detectors 98. The specialized electronics 17 for computed tomography scanning has a pipe line architecture so that the signals received from the buffers 330 are processed at high speed in real time. This ouput is stored in the

0109204

memory of the computer and in particular is stored on a hard disc storage device capable of storing data corresponding to the intensity outputs from all 1200 detectors. These so-called "views" each include as many as 1024 different data points from each detector.

Once this data has been stored, it is utilized in reconstructing a computed tomography image in accordance with procedures known in the art. Various commercial CT devices exist which utilize other reconstruction techniques for analyzing the intensity data from the detectors comprising a computed tomography scanner. The computer 16 can be readily reprogrammed to reconstruct the cross-sectional slice image using any prior art reconstruction techniques so long as the intensity values from the detector array 42 are made available in a format compatible with the reconstruction techniques used.

The present invention has been described with a degree of particularity. It should be appreciated, however, that modifications to the preferred embodiment of the invention may be made by those of ordinary skill in the art. It is the intent therefore that the application cover all modifications and/or alterations falling within the spirit or scope of the appended claims.

0109204

## Claims

1. A module(50) for mounting a plurality of detectors (98) used for sensing x-radiation intensity from a moving x-ray source (40) in a computed tomography scanner (10) of the stationary detector type, characterised in that the module (50) comprises:

a substrate (94) having a plurality of closely spaced sockets into which said plurality of detectors(98) can be positioned, said substrate (94) being curved to form a segment of a circle;

a plurality of circuit boards (96) mounted to said substrate (94), said circuit boards (96) having circuitry for receiving an analog output from said detectors (98);

connector means (69, 72) for coupling the digital output from the circuit boards (96) and transmitting said output to means for reconstructing an image from the outputs from all such circuit boards (96); and

means (60) for mounting said substrate (94) to a scanner frame (52) so that multiple numbers of said substrate when so mounted form a portion of a circle.

2. A module according to Claim 1 wherein each substrate (94) encompasses an arc of about $18^{\circ}$ so that twenty of said modules (50) when mounted to said frame (52) completely encircle a patient aperture (18) through which a patient can be positioned for computed tomography scanning.

3. A module according to Claim 1 or Claim 2 which furthermore defines an air passageway (112) so that when two such modules abut each other a flowpath for air around a patient aperture is created.

4. A module according to any preceding Claim wherein each detector (98) comprises a scintillation crystal (210) positioned next to a photodiode (212), both of which are packaged in a container (216) for shielding visible light from the detector(98).

5. A module according to any preceding Claim which further comprises circuitry mounted to said substrate (94) for powering the circuits mounted to each of said circuit boards(96).

6.     A module according to any preceding Claim where each circuit board(96) supports the electronics for converting the analog output from one detector (98) into pulses which can be counted to determine x-ray intensity.

7.     A detector module (50) for use in a computed tomography scanner, characterised in that the module (50) comprises:

  a) a housing and frame structure (74, 76, 78, 80);

  b)  a group of radiation detectors (98) carried by the structure(74, 76, 78, 80) and positioned in an arc such that when in use each detector has substantially the same source to detector relationship as others of the detectors;

  c)  electrical connectors (68, 70, 72) carried by the structure (74, 76, 78, 80) and adapted to mate with complemental connectors when positioned on the scanner;

  d)  electrical signal conduction means (96, 94) carried by the structure and coupling each of the detectors to a corresponding connector for transmitting detector outputs to said complemental connectors;

  e)  the structure including at least one module locating surface, each engagable with a corresponding and complemental scanner surface to locate the module spatially with respect to other parts of the scanner;

  f)  the structure also having a spaced pair of locating surfaces (62, 64) for locating the module relative to a contiguous pair of modules, each such locating surface being complemental with at least one surface of a contiguous module; and,

  g)  the structure including at least one attachment mechanism (60) for coactingly engaging complementary attachment mechanisms forming a part of another scanner portion.

8.     A method for sensing x-radiation intensity in computed tomography characterised by the steps of:

  mounting a plurality of x-radiation detectors (98) in closely spaced fashion onto a curved substrate (94);

  coupling each detector (98) to circuitry (96) for converting analog outputs from said detector (98) into a digital output;

mounting said circuitry (96) to said curved substrate (94);

mounting a plurality of said substrates (94) to a fixed support (52) in abutting fashion to form a circular array of detectors located along at least a segment of a circle; and

irradiating said array with x-radiation from a number of positions to thereby measure x-radiation intensity of a subject placed along the axis of said segment of a circle of said array.

9. An x-radiation detector comprising a photodiode (212) and a scintillation crystal (210) optically coupled to said photodiode, characterised in that two pin connectors (178) are coupled to said photodiode (212) for routing signals from an anode and cathode of said photodiode, a base (214) supports said photodiode (212) and defines a throughpath for said pins (178), an optical bonding agent is fixed to said base, said crystal is covered with reflective paint to shield the photodiode (212) from ambient light and a metallic shield (216) covers said base and said bonding agent to electrically shield said detector (98).

10. A method of fabricating an x-radiation detector characterised by the steps of:

mounting a photodiode (212) to a base (214) through which pins (178) extend to transmit signals from a cathode and an anode;

optically coupling a scintillation crystal (210) to said photodiode (212) with a clear adhesive and painting exterior surfaces of said crystal with a reflective paint;

shielding said photodiode (212) by affixing an opaque tape to said base (214) and said crystal (210) to prevent light from reaching said photodiode; and

electrically shielding said photodiode (212) by encapsulating said base (214) and crystal in a case (216) of low radiation absorbency.

Fig. 1

Fig. 2

Fig. 3

Fig. 4

Fig. 5

Fig. 9

Fig. 8

Fig. 7

Fig. II

0109204

0109204

Fig. 10

Fig. 12

0109204

Fig. 6

Fig. 13

Fig. 15

Fig. 14

Fig. 16

Fig. 17A

Fig. 17B

0109204

Fig. 17C